# EUROPÄISCHE PATENTSCHRIFT

(11) **EP 0 884 032 B1**
(45) Veröffentlichungstag und Bekanntmachung des Hinweises auf die Patenterteilung: **25.09.2002**
(21) Anmeldenummer: 97810367.9
(22) Anmeldetag: 12.06.1997
(51) Int. Cl.: A61F 2/34, A61F 2/38, A61F 2/40, A61B 17/17

(54) **Befestigungssystem für metallische Stützschalen**
Fastening system for shell-like supports
Système de fixation pour supports similaires à une coque

(43) Veröffentlichungstag der Anmeldung: 16.12.1998
(73) Patentinhaber: Sulzer Orthopädie AG, 6340 Baar (CH)
(72) Erfinder: Sutter, Franz, 4435 Niederdorf (CH); Jakob, Roland P., Prof. Dr., 1787 Môtier (CH); Adam, Michael, 8460 Marthalen (CH); Schoch, Roland, 6340 Baar (CH)
(74) Vertreter: Manitz, Finsterwald & Partner Gbr

(56) Entgegenhaltungen:
- DE-A- 19 542 116
- US-A- 5 534 027
- US-A- 5 571 198

## Beschreibung

Die Erfindung handelt von einem Befestigungssystem an einer metallischen Stützschale für implantierbare Gelenke mit einem stiftähnlichen metallischen Verankerungselement, welches durch die Stützschale hindurch im Knochen verankerbar ist und seinerseits einen Kopf aufweist, der in axialer Richtung des stiftähnlichen Verankerungselements an einer Schulter der Stützschale aufliegt.

Stützschalen werden an künstliche Gelenken verwendet, wenn der Werkstoff einer Lagerfläche des Gelenks in sich zu wenig steif ist, um eine dauerhafte Verankerung im Knochen zu erreichen, oder wenn die Lagerfläche austauschbar gestaltet werden soll, oder wenn der Lagerwerkstoff nicht besonders körperverträglich ist. Typische Stützschalen sind Tibiaplattformen und Aussenschalen von künstlichen Hüft- oder Schultergelenken. Der Operateur ist bei der Primärverankerung dieser Stützschalen vom Zustand des Knochens am vorgesehenen Befestigungsort abhängig und sollte daher über ein Baukastensystem verfügen, das ohne Aenderungen an der Stützschale verschiedene Befestigungsmittel zulässt.

So zeigt die EP-B-0 499 475 einen Hüftgelenkpfannenkörper, dessen Aussenschale mehrere radiale Bohrungen aufweist, in welche Stifte einsetzbar und verankerbar sind. Andere Autoren sehen Knochenschrauben vor, um eine Anpresskraft in der Richtung der Schraubenachse an der Stützschale zu erzeugen. Einen Nachteil weisen diese Systeme auf: Sie sind nicht dicht und können daher nicht verhindern, dass Abrieb durch die Bohrungen hindurch an dahinterliegende Knochenpartien gelangt.

Aufgabe der Erfindung ist es, ein Baukastensystem mit verschiedenen stiftähnlichen Verankerungselementen bereitzustellen, bei dem ein wirksames Dichtsystem die Verankerungselemente gegen die Stützschale dichtet.

Diese Aufgabe wird mit den Kennzeichen vom unabhängigen Anspruch 1 gelöst, indem zwischen Schulter und Kopf eine kreisförmige Dichtkante besteht, die bei einer vorgegebenen Axialkraft durch plastisches Fliessen eine direkte Dichtwirkung hat und das Vordringen von Verschliesspartikeln verhindert, und indem hinter dem Kopf eine Anpressschraube im Implantat angebracht ist, die die vorgegebene Axialkraft an dem Kopf erzeugt.

Die Anordnung hat den Vorteil, dass, unabhängig von einer Achsialkraft vom stiftähnlichen Verankerungselement zum Knochengewebe, eine metallische Dichtung durch plastisches Fliessen an einer Dichtkante erzeugt wird, wobei durch das Fliessen Herstelltoleranzen bezüglich Formfehler und Parallelität ausgeglichen werden, um eine flüssigkeitsdichte Verbindung zu schaffen. Diese Verbindung ist unabhängig von der Art des stiftähnlichen Verbindungselementes. Schräg eingesetzte Knochenschrauben oder glatte Positionierstifte können gleichermassen verwendet werden.

Die abhängigen Ansprüche 2 bis 10 beanspruchen vorteilhafte Weiterbildungen der Erfindung.

Je nach der Art des stiftähnlichen Verankerungselementes können Bohrlehren eingesetzt werden, die in ihrem Schwenkbereich so eingeengt sind, dass dieser dem Bereich entspricht, in dem die stiftähnlichen Verankerungselemente bei eingesetzter Stützschale einbringbar sind. Die stiftähnlichen Verankerungselemente können einen zylindrischen oder einen sphärischen Kopf aufweisen. Der aus der Stützschale vorstehende Teil kann als zylindrischer Stift, als zylindrischer Stift mit Rillen, als Wendel einer Knochenschraube, als Röhrchen mit Vor- und Rücksprüngen, als Röhrchen mit Durchbrüchen oder als Röhrchen mit Aussengewinde und Durchbrüchen ausgeführt sein.

Im folgenden wird die Erfindung anhand von Beispielen beschrieben. Es zeigen:
- Fig. 1: Schematisch einen Längsschnitt durch einen Vorsprung an einer Stützschale mit einem zylindrischen Verankerungsstift und mit einer Dichtkante an diesem Vorsprung, die auf eine Schrägfläche des Verankerungsstiftes wirkt;
- Fig. 2: schematisch einen Ausschnitt wie in Fig. 1, bei dem eine doppelte Dichtkante am Kopf des Verankerungsstiftes angebracht ist und auf eine Schulter am Vorsprung wirkt;
- Fig. 3: schematisch einen Längsschnitt durch einen Vorsprung an einer Stützschale mit einer Knochenschraube, die an ihrem Kopf dichtend geklemmt ist;
- Fig. 4: schematisch einen Längsschnitt durch einen Vorsprung an einer Stützschale mit einer Knochenschraube, die hohl ist und einen abnehmbaren Kopf aufweist;
- Fig. 5: schematisch einen Längsschnitt durch eine Stützschale mit einer Bohrlehre für eine Anordnung gemäss den Fig. 1, 2, 4;
- Fig. 6: schematisch einen Längsschnitt durch eine Stützschale mit einer Bohrlehre für eine Anordnung gemäss Fig. 3;
- Fig. 7: schematisch eine Ansicht auf eine Stützschale in Form einer Tibiaplattform mit zwei Vorsprüngen und jeweils darin verankerten Knochenschrauben; und
- Fig. 8: schematisch die Anordnung von Fig. 2 mit einer elastischen Vorspannung der Verschraubung.

In den Figuren ist ein Befestigungssystem für metallische Stützschalen 1 an implantierbaren Gelenken mit stiftähnlichen, metallischen Verankerungselementen 2 gezeigt. Die Verankerungselemente weisen einen Kopf 3 auf, der in axialer Richtung 4 an einer Schulter 5 des Implantats aufliegt. Zwischen Schulter und Kopf besteht eine kreisförmige Dichtkante 6. Durch eine Anpressschraube 7 wird am Kopf 3 eine Anpresskraft erzeugt, die so gross ist, dass durch plastisches Fliessen an der Dichtkante 6 eine metallische Dichtung erzeugt wird, die flüssigkeitsdicht ist und ein Vordringen von Abriebpartikeln aus dem inneren Bereich der Stützschale 1 verhindert.

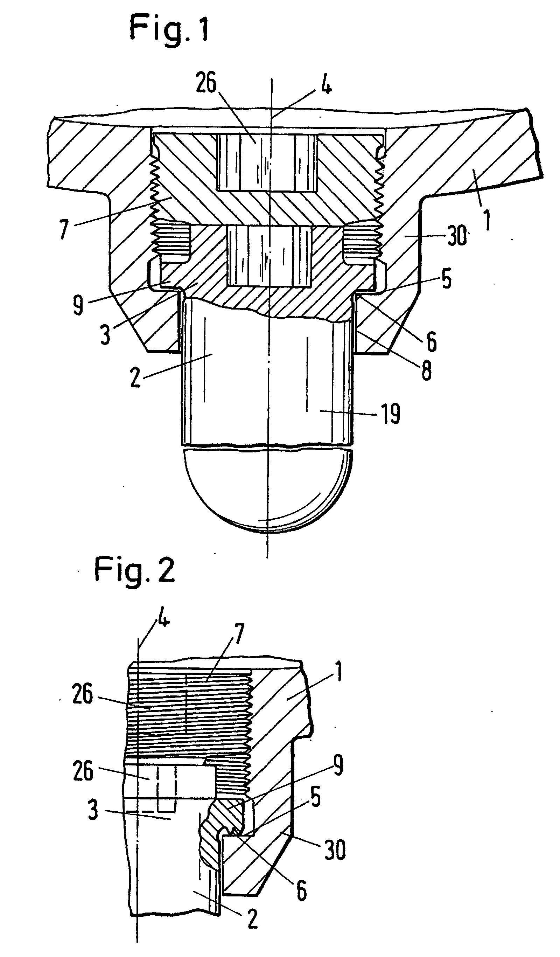

Im Beispiel von Fig. 1 ist eine Stützschale 1 mit einem Vorsprung 30 versehen, der in seiner Längsachse 4 eine Durchgangsbohrung 8 mit anschliessender Schulter 5 aufweist. Der Uebergang von der Bohrung zur Schulter ist als scharfkundige kreisförmige Dichtkante 6 ausgeführt. Ein Verankerungselement 2 ist als zylindrischer Stift 19 ausgeführt, der einen Kopf 3 mit einer Gegenschulter 9 aufweist, wobei der Uebergang vom zylindrischen Teil zur Gegenschulter 9 als Schrägfläche ausgebildet ist, die auf der Dichtkante 6 aufsitzt. Der für eine metallische Dichtung notwendige Anpressdruck wird mit einer Anpressschraube 7 erzeugt, die mit einem Gewinde in der Stützschale 1 abgestützt ist und die mit einem Werkzeug an einer Werkzeugaufnahme 26 soweit gegen den Kopf 3 geschraubt wird, bis eine vorgegebene Dichtkraft erreicht ist.

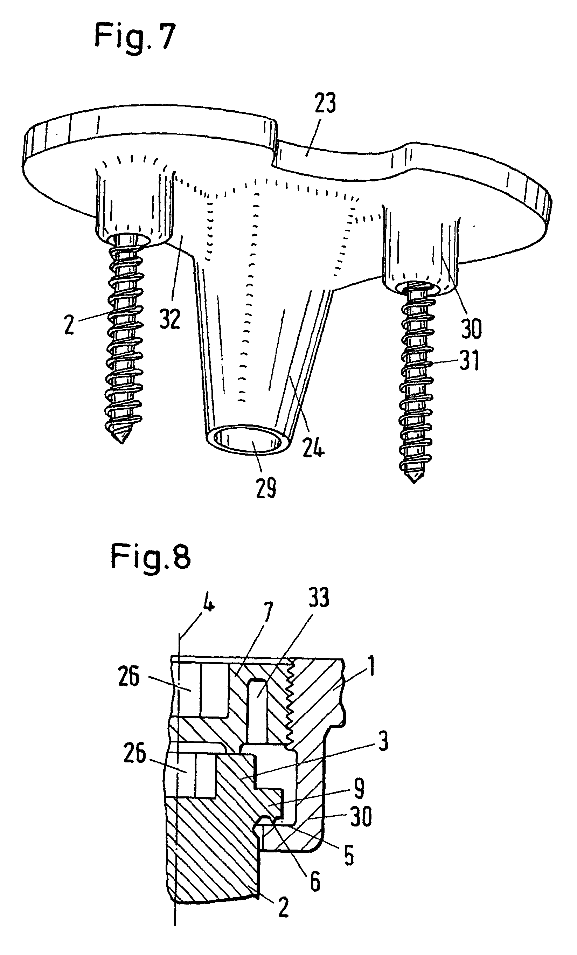

Im Beispiel von Fig. 2 sind zwei Dichtkanten 6 in der Gegenschulter 9 vom Kopf 3 angebracht und liegen auf der Schulter 5 vom Vorsprung 30 auf. Eine der Anpressschraube 7 in Fig. 1 entsprechende Schraube, die sich im gleichen Gewinde wie in Fig. 1 in der Stützschale abstützt, sorgt für die notwendige Dichtkraft. Dabei ist die Geometrie der Dichtkanten 6 auf das plastische Verhalten vom Material von Stützschale 1 und Verankerungsstift 2 abgestimmt. Die Wandstärke vom Vorsprung 30 kann dabei bewusst klein gehalten werden, um unter der Dichtkraft eine erhöhte Federwirkung in der Verschraubung zu erreichen. Damit wird nach der plastischen Verformung und Verfestigung eine Mindestdichtkraft aufrecht erhalten. In Fig. 8 ist für die gleiche Anordnung mit einer Dichtkante 6 der Vorsprung 30 mit seinem tragenden Querschnitt schlank gehalten und an der Schraube 7 ein Einstich 33 erzeugt worden, um die Federwirkung zu vergrössern.

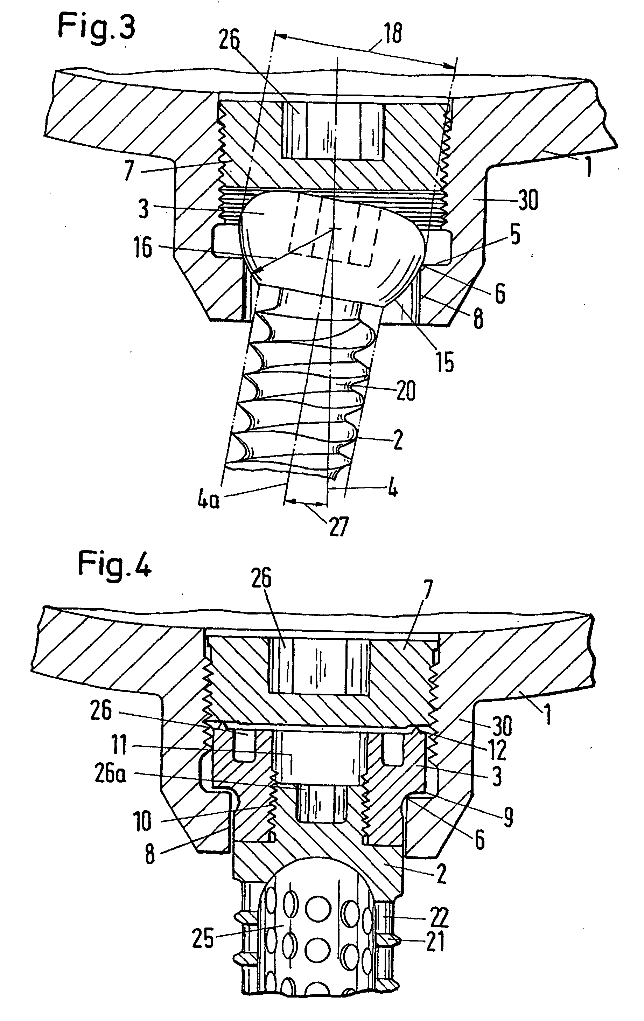

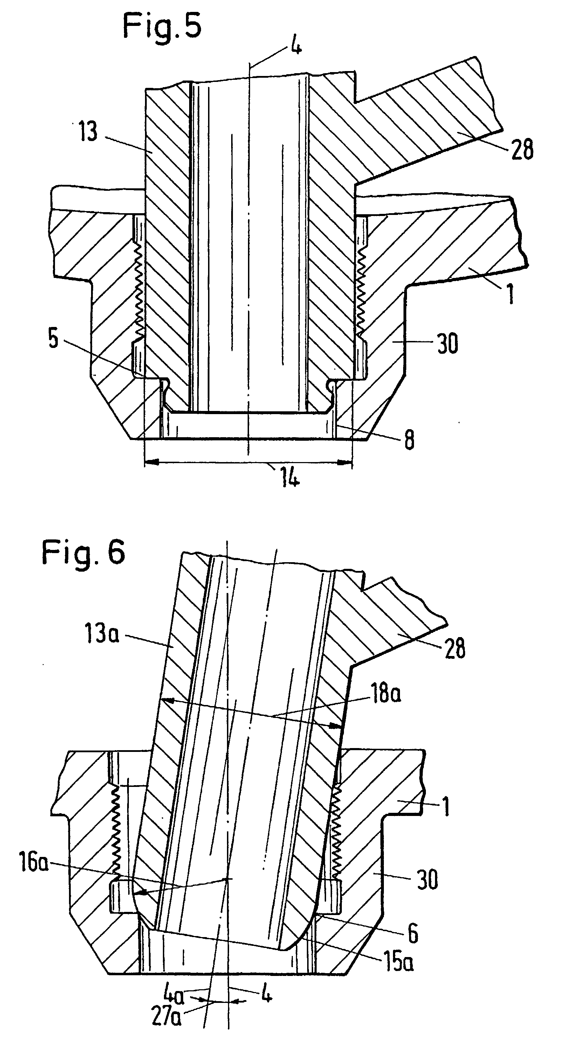

In Beispiel der Fig. 3 liegt eine Knochenschraube 20, die einen Kopf 3 mit sphärischer Auflagefläche 15 und Radius 16 besitzt, auf der Dichtkante 6 eines Vorsprungs 30 auf und zentriert sich dort. Die Schraubenachse 4a ist in eine Schrägstellung 27 aus der Längsachse 4 vom Vorsprung 30 herausgeschwenkt. Die notwendige Dichtkraft wird durch die Anpressschraube 7 erzeugt, die sich über ein Gewinde in der Stützschale 1 abstützt. Die Verspannung wird mit einem Schlüssel erzeugt, der in einer Werkzeugaufnahme 26 eingreift. Dabei ist die Schrägstellung 27 so gewählt, dass der Kopf 3 mit seinem Durchmesser 18 in die Gewindebohrung vom Vorsprung 30 aus- und eingefahren werden kann. Um diese Schrägstellung 27 bei eingesetzter Stützschale 1 nicht zu überschreiten, ist in Fig. 6 eine Bohrlehre 13a gezeigt, die eine gleiche sphärische Fläche 15a mit Radius 16a und eine zylindrische Aussenfläche mit Durchmesser 18a, der dem Durchmesser 18 vom Schraubenkopf 3 entspricht, besitzt. Die Bohrlehre 13a wird mit einem Haltearm 28 aufgesetzt und in eine gewünschte Position geschwenkt. Der Innendurchmesser der Bohrlehre ist dabei auf den Verwendungszweck des stiftähnlichen Verankerungselementes 2 abgestimmt und entspricht beispielsweise dem Aussendurchmesser eines zylindrischen Stiftes oder dem Kernlochdurchmesser für eine Knochenschraube.

Im Gegensatz dazu stützt sich die Bohrlehre 13 in Fig. 5 auf der Schulter 5 vom Vorsprung 30 ab und zentriert sich in der Durchgangsbohrung 8 oder in der Gewindebohrung vom Vorsprung 30. Mit ihr sind Bohrungen in der Richtung der Längsachse 4 möglich. Ein Haltearm 28 ermöglicht das Ansetzen und Halten während dem Bohren.

Im Beispiel der Fig. 4 ist der Kopf 3 vom Verankerungselement 2 abschraubbar. Dies ermöglicht es, den Kopf 3 und die Stützschale 1 von einem im Knochen eingewachsenen Verankerungselement 2 zu trennen. Gezeigt ist eine hohle Knochenschraube mit Aussengewinde 21 in Form eines Röhrchens 25 mit Durchbrüchen 22, die ein Einwachsen von Knochengewebe fördern sollen. Der Kopf 3 besitzt einen Durchbruch 11 und Sacklöcher 26 für einen Drehschlüssel (hier nicht gezeigt), der hohl ist, um durch den Durchbruch 11 mit einem zweiten Schlüssel an der Werkzeugaufnahme 26a des Stiftteils 2 mit einem Gegenmoment das Drehmoment beim Lösen des Kopfes 3 abzufangen. Wegen dem Durchbruch 11 besteht eine zweite metallische Dichtung in Form einer kreisförmigen Dichtkante 12 zwischen Anpressschraube 7 und dem Kopf 3.

In Fig. 7 ist eine Tibiaplattform 23 mit einem zentralen Zapfen 24 und Bohrung 29 für verschieden lange Schäfte versehen. Verstärkungsrippen 32 führen zu Vorsprüngen 30, aus denen Knochenschrauben 31 herausragen, die gemäss den vorangehenden Beispielen mit einer metallischen Dichtung an den Vorsprüngen 30 verankerbar sind.

Die hier gezeigten Beispiele lassen sich grundsätzlich auch als Befestigungssysteme in der Traumatologie für metallische Knochenplatten verwenden, da die erzeugte metallische Dichtung bezüglich der Uebertragung von Biegemomenten von einem plattenähnlichen Element auf stiftförmige im Knochen verankerte Elemente grosse Kräfte übertragen kann. Diese Eigenschaft, die diese Verbindung bezüglich Dichtheit gegen Biegemomente unempfindlich macht, ist zwangsläufig auch eine gute Verankerung zwischen einer Knochenplatte und einem stiftähnlichen Element wie zum Beispiel einer Knochenschraube. Eine mit den Figuren 3 und 6 gezeigte Ausführung lässt sich besonders gut in Operationstechniken anwenden, bei denen eine einen Knochen verstärkende Platte unter den Muskeln durchgeschoben und an ihren Enden mit dem Knochen durch Knochenschrauben verbunden wird. Die plastische Deformation in der metallischen Dichtung erzeugt eben auch einen Formschluss für eine bessere Uebertragung der Kräfte.

## Patentansprüche

1. Befestigungssystem an einer metallischen Stützschale (1) für implantierbare Gelenke mit einem stiftähnlichen metallischen Verankerungselementen (2), welches durch die Stützschale hindurch im Knochen verankerbar ist und seinerseits einen Kopf (3) aufweist, der in axialer Richtung (4) des stiftähnlichen Verankerungselements (2) an einer Schulter (5) der Stützschale aufliegt, **dadurch gekennzeichnet, dass** zwischen Schulter (5) und Kopf (3) eine kreisförmige Dichtkante (6) besteht, die bei einer vorgegebenen Axialkraft durch plastisches Fliessen eine direkte Dichtwirkung hat und das Vordringen von Verschleisspartikeln verhindert, und dass hinter dem Kopf (3) eine Anpressschraube (7) im Implantat angebracht ist, die die vorgegebene Axialkraft an dem Kopf (3) erzeugt.

2. Befestigungssystem nach Anspruch 1, **dadurch gekennzeichnet, dass** die Schulter (5) gerade und im rechten Winkel zur Achse (4) des stiftähnlichen Verankerungselementes (2) steht, um an einer Durchgangsbohrung (8) eine Dichtkante (6) für einen sphärischen Kopf (3) eines Verankerungselementes (2) zu bilden.

3. Befestigungssystem nach Anspruch 1 oder 2, **dadurch gekennzeichnet, dass** der Kopf (3) eine gerade, ringförmige Gegenschulter (9) mit mindestens einer vorstehenden Dichtkante (6) aufweist.

4. Befestigungssystem nach Anspruch 3, **dadurch gekennzeichnet, dass** Kopf (3) und Gegenschulter (9) mit einer lösbaren Schraubverbindung (10) am stiftähnlichen Verankerungselement (2) befestigbar ist.

5. Befestigungssystem nach Anspruch 4, **dadurch gekennzeichnet, dass** der lösbare Kopf (3) einen mittleren Durchbruch (11) für ein Werkzeug und auf seiner Oberseite eine weitere kreisförmige Dichtkante (12) zum Dichten gegen die Anpressschraube (7) aufweist.

6. Befestigungssystem nach einem der Ansprüche 3 bis 5, **dadurch gekennzeichnet, dass** eine Bohrlehre (13) mit einem Aussendurchmesser (14), der dem Innendurchmesser vom Gewinde der Anpressschraube entspricht, am Implantat (1) aufsteckbar ist, um eine dem stiftähnlichen Verankerungselement (2) entsprechende Bohrung herzustellen.

7. Befestigungssystem nach Anspruch 1 oder 2, **dadurch gekennzeichnet, dass** der Kopf (3) eine sphärische Fläche (15) mit Radius (16) zur Schulter aufweist (5), die nach innen über die Schulterkante (6) übersteht, um einerseits den Kopf (3) an der Schulterkante (6) zu zentrieren und um andererseits die Schulterkante als kreisförmige Dichtkante zu verwenden.

8. Befestigungssystem nach Anspruch 7, **dadurch gekennzeichnet, dass** der Durchmesser der Schulterkante (6) grösser als der Durchmesser (17) vom vorstehenden stiftähnlichen Teil ist, um das stiftähnliche Verankerungselement (2) in einer geschwenkten Achse (4a) einführbar und verankerbar zu machen.

9. Befestigungssystem nach Anspruch 7 oder 8, **dadurch gekennzeichnet, dass** eine Bohrlehre (13a) mit einer sphärischen Abstützfläche (15a), die der sphärischen Fläche (15) vom Kopf (3) entspricht, versehen ist und einen Aussendurchmesser (18a) besitzt, der kleiner als der Innendurchmesser vom Gewinde der Anpressschraube (7) ist, um im möglichen Schwenkbereich des stiftähnlichen Verankerungselements (2) Vorbohrungen anbringen zu können.

10. Befestigungssystem nach einem der Ansprüche 1 bis 9, **dadurch gekennzeichnet, dass** der vorstehende Teil vom stiftähnlichen Verankerungselement (2) als zylindrischer Stift (19), als zylindrischer Stift (19) mit Längsrillen, als zylindrischer Stift (19) mit Querrillen, als Wendel einer Knochenschraube (20) als Röhrchen (25) mit Vor- und Rücksprüngen, als Röhrchen (25) mit Durchbrüchen (22) oder als Röhrchen mit Aussengewinde (21) und Durchbrüchen (22) ausgeführt ist.

11. Implantat mit einem Befestigungssystem nach einem der Ansprüche 1 bis 10.

## Claims

1. Mounting system at a metallic support shell (1) for implantable joints with a pin-like metallic anchoring element (2) which can be anchored in the bone while passing through the support shell and which itself has a head (3) which lies on a shoulder (5) of the support shell in the axial direction (4) of the pin-like anchoring element (2), **characterised in that** between the shoulder (5) and the head (3) there is a circular sealing edge (6) which has a direct sealing action due to plastic flow at a predetermined axial force and prevents the intrusion of abraded particles; and **in that** a contact pressure generating screw (7) is inserted into the implant behind the head (3) and produces the predetermined axial force at the head (3).

2. Mounting system in accordance with claim 1, **characterised in that** the shoulder (5) stands straight and at a right angle to the axis (4) of the pin-like anchoring element (2) in order to form a sealing edge (6) at a through-going bore (8) for a spherical head (3) of an anchoring element (2).

3. Mounting system in accordance with claim 1 or claim 2, **characterised in that** the head (3) has a straight, ring-shaped counter-shoulder (9) with at least one protruding sealing edge (6).

4. Mounting system in accordance with claim 3, **characterised in that** the head (3) and the counter-shoulder (9) can be fastened to the pin-like anchoring element (2) with a releasable screw connection (10).

5. Mounting system in accordance with claim 4, **characterised in that** the releasable head (3) has a central aperture (11) for a tool and a further circular sealing edge (12) at its upper side for sealing against the contact pressure generating screw (7).

6. Mounting system in accordance with one of the claims 3 to 5, **characterised in that** a drilling jig (13) with an outer diameter (14) which corresponds to the inner diameter of the thread of the contact pressure generating screw can be pushed onto the implant (1) in order to produce a bore corresponding to the pin-like anchoring element (2).

7. Mounting system in accordance with claim 1 or claim 2, **characterised in that** the head (3) has a spherical surface (15) with a radius (16) to the shoulder (5) which projects inwardly over the shoulder edge (6) in order, on the one hand, to centre the head (3) at the shoulder edge (6) and, on the other hand, to be able to use the shoulder edge as a circular sealing edge.

8. Mounting system in accordance with claim 7, **characterised in that** the diameter of the shoulder edge (6) is greater than the diameter (17) of the protruding pin-like part in order to be able to introduce and anchor the pin-like anchoring element (2) with a pivotally deflected axis (4a).

9. Mounting system in accordance with claim 7 or claim 8, **characterised in that** a drilling jig (13a) with a spherical support surface (15a) which corresponds to the spherical surface (15) of the head (3) is provided and has an outer diameter (18a) which is smaller than the inner diameter of the thread of the contact pressure generating screw (7) in order to be able to make pre-bores in the possible range of pivoting of the pin-like anchoring element (2).

10. Mounting system in accordance with one of the claims 1 to 9, **characterised in that** the projecting part of the pin-like anchoring element (2) is executed as a cylindrical pin (19), as a cylindrical pin (19) with longitudinal grooves, as a cylindrical pin (19) with transverse grooves, as the spiral thread of a bone screw (20), as a tubule (25) with projections and recesses, as a tubule (25) with apertures (22) or as a tubule with an outer thread (21) and apertures (22).

11. Implant with a mounting system in accordance with one of the claims 1 to 10.

## Revendications

1. Système de fixation à une coque de support métallique (1) pour des articulations aptes à être implantées avec un élément d'ancrage métallique (2) semblable à une broche qui peut être ancré à travers la coque de support dans l'os et qui présente une tête (3) qui s'applique dans la direction axiale (4) de l'élément d'ancrage (2) semblable à une broche à un épaulement (5) de la coque de support, **caractérisé en ce qu'**il existe entre l'épaulement (5) et la tête (3) une arête d'étanchéité circulaire (6) qui, lors d'une force axiale prédéfinie produit par fluage plastique un effet étanche direct et empêche la pénétration de particules d'usure, et **en ce qu'**il est disposé derrière la tête (3) une vis d'application (7) dans l'implant qui produit la force axiale prédéfinie à la tête (3).

2. Système de fixation selon la revendication 1, **caractérisé en ce que** l'épauleme (5) s'étend d'une manière rectiligne et à angle droit à l'axe (4) de l'élément d'ancrage (2) semblable à une broche pour former à un perçage traversant (8) une arête d'étanchéité (6) pour une tête sphérique (3) d'un élément d'ancrage (2).

3. Système de fixation selon la revendication 1 ou 2, **caractérisé en ce que** la tête (3) présente un contre-épaulement annulaire droit (9) avec au moins une arête d'étanchéité saillante (6).

4. Système de fixation selon la revendication 3, **caractérisé en ce que** la tête (3) et le contre-épaulement (9) peuvent être fixés par un assemblage par vissage amovible (10) à l'élément d'ancrage (2) semblable à une broche.

5. Système de fixation selon la revendication 4, **caractérisé en ce que** la tête amovible (3) présente un perçage médian (11) pour un outil et sur son côté supérieur une autre arête d'étanchéité circulaire (12) en vue d'un étanchement relativement à la vis d'application (7).

6. Système de fixation selon l'une des revendications 3 à 5, **caractérisé en ce qu'**un gabarit de perçage (13) d'un diamètre extérieur (14) qui correspond au diamètre intérieur du filetage de la vis d'application, peut être placé sur l'implant (1) pour établir un perçage correspond à l'élément d'ancrage (2) semblable à une broche.

7. Système de fixation selon la revendication 1 ou 2, **caractérisé en ce que** la tête (3) présente une face sphérique (15) avec un rayon (16) vers l'épaulement (5) qui fait saillie vers l'intérieur sur l'arête d'épaulement (6) pour centrer d'une part la tête (3) à l'arête d'épaulement (6) et pour utiliser d'autre part l'arête d'épaulement comme arête d'étanchéité circulaire.

8. Système de fixation selon la revendication 7, **caractérisé en ce que** le diamètre de l'arête d'épaulement (6) est plus grand que le diamètre (17) de la partie saillante semblable à une broche pour que l'élément d'ancrage (2) semblable à une broche puisse être inséré et ancré dans un axe pivoté (4a).

9. Système de fixation selon la revendication 7 ou 8, **caractérisé en ce qu'**un gabarit de perçage (13a) est pourvu d'une face d'appui sphérique (15a) qui correspond à la face sphérique (15) de la tête (3) et qui possède un diamètre extérieur (18a) qui est plus petit que le diamètre intérieur du filetage de la vis d'application (7) pour pouvoir ménager dans la zone de pivotement possible de l'élément d'ancrage (2) semblable à une broche des pré-perçages.

10. Système de fixation selon l'une des revendications 1 à 9, **caractérisé en ce que** la partie saillante de l'élément d'ancrage (2) semblable à une broche est réalisée comme broche cylindrique (19), comme broche cylindrique (19) avec des rainures longitudinales, comme broche cylindrique (19) avec des rainures transversales, comme hélice d'une vis à os (20), comme petit tube (25) avec des saillies et des retraits, comme petit tube (25) avec des perçages (22) ou comme petit tube avec un filetage extérieur (21) et des perçages (22).

11. Implant avec un système de fixation selon l'une des revendications 1 à 10.
